# FASCICULE DE BREVET EUROPEEN

(11) **EP 0 755 925 B1**
(45) Date de publication et mention de la délivrance du brevet: **11.11.1998**
(21) Numéro de dépôt: 96401662.0
(22) Date de dépôt: 25.07.1996
(51) Int. Cl.: C07D 213/38, C07D 257/04, A61K 31/44, A61K 7/48

(54) **Nouveaux dérivés de N,N'-di(aralkyl)-N,N'-di(2-azaaralkyl)alkylène diamine et leur utilisation dans des compositions pharmaceutiques et cosmétiques**
N,N'-Di(aralkyl)-N,N'-di(2-azaaralkyl)alkylendiamin-Derivate und ihre Verwendung in pharmazeutischen und kosmetischen Zusammensetzungen
N,N'-di(aralkyl)-N,N'-di(2-azaaralkyl)alkylene-diamine derivatives and their use in pharmaceutical and cosmetic compositions

(30) Priorité: 26.07.1995 FR 9509118
(43) Date de publication de la demande: 29.01.1997
(73) Titulaire: L'OREAL, 75008 Paris (FR)
(72) Inventeur: Galey, Jean-Baptiste, 93600 Aulnay-sous-Bois (FR); Dumats, Jacqueline, 93420 Villepinte (FR)
(74) Mandataire: Stalla-Bourdillon, Bernard

(56) Documents cités:
- WO-A-94/11338
- CHEMICAL ABSTRACTS, vol. 123, no. 12, 18 Septembre 1995 Columbus, Ohio, US; abstract no. 159300c, B. RIEGER ET AL.: "Synthesis of chiral and C2-symmetric iron(II) and cobalt(II) complexes bearing a new tetradentate amine ligand system." page 1125; XP002017481 & JOURNAL OF ORGANOMETALLIC CHEMISTRY, vol. 497, no. 1-2, 26 Juillet 1995, LAUSANNE, pages 73-79,

## Description

La présente invention a pour objet de nouveaux dérivés de N,N'-di(aralkyl) N,N'-di(2-azaaralkyl) alkylène diamine qui trouvent une utilisation dans des compositions pharmaceutiques et cosmétiques en vue de protéger l'organisme contre le stress oxydant.

Le stress oxydant est défini pour un certain nombre de situations physiopathologiques et pathologiques comme un déséquilibre de la balance antioxydant-prooxydant. Ce déséquilibre se traduit notamment par des processus oxydatifs non contrôlés au sein des tissus vivants qui mettent en jeu des radicaux libres oxygénés et conduisent notamment à la formation de dégâts oxydatifs sur les molécules et macromolécules biologiques.

Un certain nombre de situations physiopathologiques provoquent, favorisent, accompagnent ou sont la conséquence directe d'un stress oxydant. Il s'agit notamment de l'inflammation, du vieillissement, de l'exposition aux ultra-violets et aux rayonnement ionisants, de la carcinogénèse, des situations d'ischémie reperfusion, de la toxicité et/ou du mode d'action de certains médicaments.

Lors d'un stress oxydant, il a été montré que du fer était libéré de ses sites de stockage usuels comme la ferritine et pouvait alors participer à certaines réactions et notamment aux réactions de Fenton et Haber-Weiss dont résulte la formation des radicaux hydroxyles, radicaux connus pour être responsables de nombreux dommages oxydatifs.

Il a été proposé en vue de se protéger contre les radicaux hydroxyles d'utiliser des molécules telles que le D-mannitol, l'acide benzoïque, ou le DMSO, ceux-ci étant capables de piéger les radicaux hydroxyles. Toutefois les radicaux hydroxyles sont particulièrement réactifs et il convient d'utiliser des quantités relativement importantes de ces piégeurs de manière à entrer en compétition avec toutes les molécules biologiques, cibles potentielles des radicaux hydroxyles ce qui n'est pas sans présenter certains inconvénients du fait des problèmes de toxicité de ces piégeurs.

Par ailleurs, en vue de se protéger contre les radicaux hydroxyles, il a été proposé d'utiliser des chélateurs du fer, notamment la deferoxamine ou l'acide diéthylène triamine pentaacétique (DTPA), ceci en vue d'empêcher le fer de participer aux réactions de Fenton et Haber-Weiss.

Néanmoins il s'est avéré que la plupart des ces chélateurs sont relativement toxiques, ceux-ci pouvant interférer avec le métabolisme du fer et chelater le fer des sites actifs de certains enzymes ou d'hémoprotéines comme l'hémoglobine.

Dans la demande de brevet WO 94/11338, il a été proposé d'utiliser certains composés susceptibles de former des complexes avec le fer dont les constantes de stabilité sont faibles, ce qui diminue par conséquent les risques de toxicité associés à leur utilisation.

Après de nouvelles recherches sur des substances agissant selon un mécanisme comparable, on a réussi la synthèse d'une nouvelle classe de composés présentant une meilleure biodisponibilité, ce qui jusqu'à présent n'avait pu être obtenu avec les complexants du fer de l'art antérieur.

Il s'est par ailleurs avéré que ces nouveaux composés formaient avec le fer des complexes dont les constantes d'association sont plus faibles que celles des chélateurs connus, tels que la deferoxamine. En outre, dans la mesure où, pour des raisons thermodynamiques, ils ne peuvent déplacer le fer de la transferrine, les risques toxicologiques sont donc diminués.

De plus le potentiel d'oxydo-réduction des complexes de fer des composés selon l'invention est tel qu'ils sont réductibles par les réducteurs physiologiques et capables sous forme réduite, de réagir avec le peroxyde d'hydrogène pour former des radicaux hydroxyles qui sont aussitôt piégés par un processus d'hydroxylation aromatique intramoléculaire de façon quasi stoechiométrique, avant qu'ils ne puissent attaquer d'autres molécules.

Après hydroxylation intramoléculaire, ces composés possèdent alors un résidu hydroxy aromatique qui peut occuper un cinquième ou un sixième site de coordination du fer. Etant donné la grande affinité des résidus phénolates pour le fer ferrique, ceci a pour conséquence d'augmenter la stabilité des complexes de plusieurs ordres de grandeur et d'empêcher la participation ultérieure du fer à la catalyse de dommages oxydatifs.

La présente invention a donc pour objet à titre de composés nouveaux, des dérivés de N,N'-di(aralkyl) N,N'-di(2-azaaralkyl) alkylène diamine pouvant être représentés par la formule générale suivante : 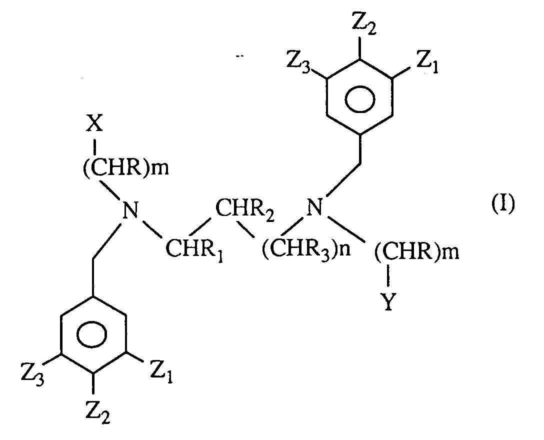 dans laquelle :
n est 0, 1 ou 2,
m est 1, 2 ou 3,
R, R₁, R₂ et R₃, identiques ou différents, représentent un atome d'hydrogène ou un radical alkyle, linéaire ou ramifié, en C₁-C₄,
R₁ et R₂ ou R₂ et R₃ pris ensemble pouvant former un cycle cyclopentyle ou cyclohexyle,
X et Y identiques ou différents représentent un hétérocycle aromatique choisi parmi les radicaux 2-pyridyle, 2-pyrimidyle, 4-pyrimidyle, 1,3,5-triazin-2-yle, 2-imidazolyle, 2-pyrrolyle, 2-tétrazolyle, 4-thiazolyle, 2-méthyl-4-thiazolyle et 2-benzoimidazolyle,
Z₁, Z₂ et Z₃, identiques ou différents, représentent un atome d'hydrogène, un radical alkyle, linéaire ou ramifié, en C₁-C₄, le radical -OR₄ ou -NR₄R'₄,
R₄ et R'₄ représentant un atome d'hydrogène ou un radical alkyle, linéaire ou ramifié, en C₁-C₄,
   et leurs sels et complexes métalliques.

Par radical alkyle, linéaire ou ramifié en C₁-C₄ on doit entendre des radicaux tels que méthyle, éthyle, isopropyle et tert-butyle.

Selon une forme particulièrement préférée des composés selon l'invention, les radicaux Z₁, Z₂ et Z₃ sont des groupes électro donneurs et de préférence représentent au moins un groupe méthoxy.

Parmi les sels des composés de formule (I) on peut notamment citer les sels d'addition d'un acide minéral tel que l'acide sulfurique, l'acide chlorhydrique, l'acide nitrique ou l'acide phosphorique.

Parmi les complexes on peut citer ceux formés par addition de chlorure de zinc ou de chlorure de calcium.

A titre d'exemple on peut citer en tant que composés représentatifs des composés de formule (I) les suivants :
N,N'-bis-(3,4,5-triméthoxybenzyl) N,N'-bis[1-(2-tétrazolyl) éthyl] éthylènediamine,
N,N'-bis-(3,4,5-triméthoxybenzyl) N,N'-bis-(2-pyridyl méthyl) éthylènediamine,
N,N'-bis-(2-méthyl-thiazol-4-ylméthyl)-N-N'-bis-(3,4,5-triméthoxybenzyl) éthylènediamine,
N,N'-bis-(1H-benzoimidazol-2-ylméthyl)-N,N'-bis-(3,4,5-triméthoxybenzyl) éthylènediamine.

La présente invention a également pour objet le procédé de préparation des composés de formule générale (I) qui peut être représenté par le schéma réactionnel suivant :

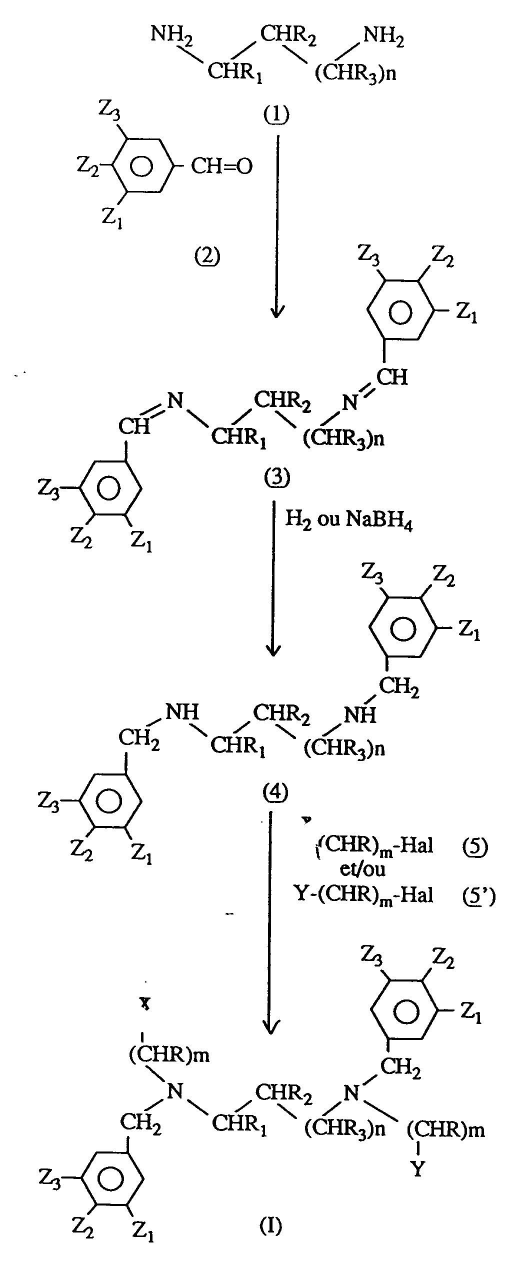

Le procédé selon l'invention consiste à faire réagir une alkylène diamine de formule (1) avec un aldéhyde aromatique de formule (2) en milieu solvant organique à une température inférieure au point d'ébullition du solvant. La diimine (3) obtenue, isolée ou non, est ensuite réduite en présence de borohydrure de sodium ou par hydrogénation catalytique pour conduire à la diamine de formule (4).

La diamine (4) est ensuite traitée en milieu basique, par exemple en présence de soude, à l'aide d'un composé halogéné de formule (5) et/ou (5'), (Hal étant de préférence un atome de chlore ou de brome).

Après extraction et séchage, on obtient alors le composé de formule générale (I).

La présente invention a en outre pour objet une composition cosmétique ou pharmaceutique contenant au moins un composé de formule (I) ou l'un de ses sels ou complexes métalliques dans un véhicule cosmétiquement ou pharmaceutiquement acceptable.

Dans ces compositions, le composé actif de formule (I) est généralement présent en une proportion de 0,001 à 10% en poids par rapport au poids total de la composition.

Les compositions cosmétiques peuvent se présenter sous diverses formes conventionnelles telles que sous forme d'onguent, de crème, de pommade, de gel, de spray, de lotion, d'émulsion ou de dispersion vésiculaire.

Dans les compositions selon l'invention, il a été constaté par ailleurs que le composé de formule (I) jouait un rôle important par son action antioxydante et permettait ainsi de les protéger de l'oxydation.

Lorsque le composé de formule (I) est utilisé dans le cadre d'un traitement pharmaceutique, les formes d'administration peuvent être par voie orale, topique ou parentérale, le support pharmaceutiquent acceptable étant fonction de la forme d'administration choisie. Les doses d'administration sont généralement comprises entre 1 mg et 1000 mg/kg/jour.

Les compositions pharmaceutiques selon l'invention sont tout particulièrement destinées à traiter les situations de stress oxydant liées à certains états pathologiques et notamment les maladies neurodégénératives telles que par exemple la maladie de Parkinson, les situations inflammatoires chroniques, le syndrome d'ischémie reperfusion, la toxicité de certains médicaments tels que certains xénobiotiques et les surcharges en fer.

Dans les compositions selon l'invention le composé de formule (I) peut, selon une forme de réalisation préférée, être associée à au moins une autre substance active (ou une autre substance anti-radicaux-libres). Ces substances peuvent être choisies plus particulièrement parmi :
- les antilipoperoxydants comme la vitamine E, le trolox, le BHT (butylhydroxytoluène),
- les réducteurs biologiques comme le glutathion réduit et ses dérivés, la vitamine C et ses dérivés,
- les quencheurs d'oxygène singulet comme le β-carotène,
- les systèmes capables de décomposer le peroxyde d'hydrogène et notamment des enzymes comme la catalase ou des peroxydases en présence de leurs co-substrats,
- les systèmes de protection contre l'anion superoxyde comme la superoxyde dismutase (SOD) ou des SOD-like tels que le complexe Mn-desferal ou le di-isopropyl salicylate de cuivre,
- les systèmes capables de décomposer les hydroperoxydes organiques comme la glutathion peroxydase ou des systèmes à base de sélénium.

Le composé de formule (I) et les substances actives ou substances antiradicaux libres telles que définies ci-dessus, peuvent être associés au sein de la même composition ou être appliqués séparément.

Les exemples ci-après sont donnés en vue d'illustrer le procédé de préparation des composés de formule (I) et leur utilisation dans les domaines pharmaceutique et cosmétique.

### EXEMPLES DE PREPARATION DES COMPOSES

### EXEMPLE 1 : Préparation de la N,N'-bis-(3,4,5-triméthoxybenzyl) N,N'-bis-(2-pyridyl méthyl) éthylènediamine

1) Dans un tricol de 1 litre, on met en suspension, à température ambiante 50 g de 3,4,5-triméthoxybenzaldéhyde dans 300 ml de méthanol. On chauffe à 40°C pour obtenir une dissolution complète et on ajoute goutte à goutte 8,5 ml d'éthylène diamine. On laisse alors revenir à température ambiante. Le milieu réactionnel jaune limpide à la fin de l'addition de la diamine devient rapidement hétérogène avec précipitation de la diimine. On laisse encore sous agitation pendant une heure à température ambiante avant de refroidir à 5°C. Le précipité est collecté par filtration sur verre fritté et lavé abondamment par du méthanol froid puis remis en suspension dans du méthanol glacé et filtré pour éliminer toute trace d'aldéhyde résiduel. Le précipité blanc (49 g) est ensuite séché sous vide au dessicateur. F=148°C.
2) Dans un tricol de 1 litre on met en suspension à température ambiante 20 g de la diimine obtenue ci-dessus en 1) dans 400 ml d'éthanol absolu. On ajoute alors 2,27g de borohydrure de sodium en pastilles et on chauffe à 55-60°C. Après environ 45 minutes, le milieu réactionnel est devenu limpide. On maintient 2 h à 55-60°C puis on laisse revenir à température ambiante doucement et on hydrolyse avec une solution aqueuse d'HCl 6N jusqu'à pH<1. Le milieu réactionnel devient jaune, puis un précipité apparait rapidement. On refroidit alors à +5°C pendant 1h environ sous faible agitation. Le précipité est filtré sur verre fritté et lavé par de l'éthanol absolu avant d'être séché sous vide au dessicateur. Le dichlorhydrate de N,N'-bis-(3,4,5-triméthoxybenzyl) éthylènediamine obtenu est utilisé tel quel pour la dernière étape.
3) On solubilise 2 g de dichlorhydrate de N,N'-bis-(3,4,5-trimethoxybenzyl) éthylènediamine obtenue ci-dessus en 2) dans 30 ml d'eau. La solution est amenée à pH 11 par de la lessive de soude. On additionne en une fois une solution de 1,35 g de chlorure de 2-picolyle dans 15 ml d'eau. On chauffe le mélange 4 h à 40°C tout en maintenant le pH entre 10 et 11 par addition de lessive de soude. Le milieu est alors extrait par 3 fois 25 ml de dichlorométhane puis la phase organique est lavée à l'eau saturée de NaCl. Après séchage et évaporation à sec, on obtient 1,6 g d'une huile brune. Cette huile est purifiée par chromatographie sur colonne de silice (éluant dichlorométhane/méthanol). On obtient 1,4 g de N,N'-bis-(3,4,5- trimethoxybenzyl) N, N'-bis-(2-pyridyl méthyl) éthylènediamine sous forme d'huile (Rdt=57%). Celle-ci a alors été cristallisée pour donner naissance à un solide blanc de point de fusion = 106°C dont le spectre RMN ¹H (400 MHz), le spectre de masse et l'analyse élémentaire sont conformes à la structure attendue.

| | C | H | N | O |
|---|---|---|---|---|
| Calculé % (avec 0,2 H₂O) | 67,37 | 7,00 | 9,25 | 16,38 |
| Trouvé % | 66,95 | 6,96 | 9,19 | 16,26 |

### EXEMPLE 2 : Préparation de la N,N'-bis-(2-méthyl-thiazol-4-ylméthyl)-N,N' -bis-(3,4,5-triméthoxybenzyl) éthylènediamine

Pour la préparation de ce composé, on réalise tout d'abord les deux mêmes premières étapes 1) et 2) de l'exemple 1.

3) On solubilise 1,5 g de dichlorhydrate de N,N'-bis-(3,4,5-triméthoxybenzyl) éthylènediamine dans 30 ml d'un mélange éthanol/eau 1:1. La solution est amenée à pH 11 par de la lessive de soude. On additionne en une fois une solution de 1,25 g de chlorhydrate de 4-chlorométhyl-2-méthylthiazole en solution dans 10 ml d'eau. On chauffe le mélange 5 heures à 45°C tout en maintenant le pH entre 10 et 11 par addition de lessive de soude. Après refroidissement, on évapore sous vide l'éthanol et on acidifie à pH 1 avec HCl concentré. On extrait alors par 3 x 25 ml de dichlorométhane puis la phase organique est séchée et on évapore ensuite à sec. On obtient une huile qu'on reprend par 10 ml d'éthanol + 0,5 ml d'HCl concentré. Un précipité apparait qu'on filtre et lave à l'éther éthylique. Le solide est alors recristallisé dans 20 ml d'isopropanol contenant 1 % d'eau. On obtient ainsi 600 mg d'une poudre blanche de point de fusion = 190°C dont le spectre RMN du proton (400 MHz) et l'analyse élémentaire sont conformes à la structure attendue (sous forme de dichlorhydrate).

| | C | H | N | O | S | Cl |
|---|---|---|---|---|---|---|
| Calculé % (avec 0,35 H₂O) | 53,24 | 6,20 | 7,76 | 14,09 | 8,87 | 9,84 |
| Trouvé % | 53,53 | 6,19 | 7,75 | 14,18 | 8,66 | 9,82 |

### EXEMPLE 3 : Préparation de la N,N'-bis-(1H-benzoimidazol-2-ylméthyl)-N,N'-bis-(3,4,5-triméthoxybenzyl) éthylènediamine

Pour la préparation de composé, on réalise tout d'abord les deux mêmes premières étapes 1) et 2) de l'exemple 1.

3) On solubilise 1,5 g de dichlorhydrate de N,N'-bis-(3,4,5-triméthoxybenzyl) éthylènediamine dans 30 ml d'un mélange éthanol/eau 1:1. La solution est amenée à pH 11 par de la lessive de soude. On additionne en une fois une solution de 1,1 g de 2-chlorométhyl benzimidazole en solution dans 30 ml d'éthanol. On chauffe le mélange 5 heures à 45°C tout en maintenant le pH entre 10 et 11 par addition de lessive de soude. Après refroidissement, on évapore sous vide l'éthanol et on refroidit le milieu à 0°C. Le précipité formé est filtré et lavé à l'eau. Le solide est repris dans 15 ml d'eau + 0,5 ml d'HCl concentré puis filtré et lavé à l'eau. Le solide est alors recristallisé dans 10 ml d'un mélange eau/éthanol 98:2. On obtient 350 mg d'une poudre blanche de point de fusion = 150°C (dec.) dont le spectre RMN du proton (400 MHz) et l'analyse élémentaire sont conformes à la structure attendue (sous forme de dichlorhydrate).

| | C | H | N | O | Cl |
|---|---|---|---|---|---|
| Calculé % (avec 3,5 H₂O) | 55,88 | 6,50 | 10,29 | 18,63 | 8,70 |
| Trouvé % | 55,46 | 6,61 | 10,31 | 18,75 | 8,36 |

### EXEMPLE DE COMPOSITION PHARMACEUTIQUE

### EXEMPLE A

On prépare selon l'invention une suspension buvable en procédant au mélange des ingrédients suivants :
- N,N'-bis-(3,4,5-triméthoxybenzyl) N,N'-bis-(2-pyridyl méthyl)-éthylènediamine (composé de l'exemple 1) 0,10 g
- Ethanol à 90 % 1,00 g
- Sorbitol à 70 % 0,50 g
- Saccharinate de sodium 0,01 g
- Conservateur 0,04 g
- Arôme qs
- Eau purifiée qsp 5 ml

Cette composition buvable administrée 1 à 2 fois par jour pendant au moins 3 à 5 semaines permet de traiter de façon efficace la plupart des maladies neurodégénératives.

Dans cette solution buvable, le composé selon l'exemple 1 peut être avantageusement remplacé par la même quantité de l'un des composés des exemples 2 et 3.

### EXEMPLE DE COMPOSITION COSMETIQUE

### EXEMPLE B

On prépare selon l'invention une émulsion huile-dans-eau en procédant au mélange des ingrédients suivants :
- N,N'-bis-(3,4,5-triméthoxybenzyl) N,N'-bis(2-pyridyl méthyl)-éthylènediamine (composé de l'exemple 1) 0,50 %
- Huile de jojoba 13,00 %
- Sorbate de potassium 0,30 %
- Cyclopentadiméthylsiloxane 10,00 %
- Alcool stéarylique 1,00 %
- Acide stéarique 4,00 %
- Stéarate de polyéthylène glycol 3,00 %
- Vitamine E 1,00 %
- Glycérol 3,00 %
- Conservateur 0,05 %
- Eau qsp 100,00 %

Cette composition appliquée régulièrement au moins une fois par jour, de préférence le soir, permet de prévenir de façon particulièrement significative le vieillissement cutané. Par ailleurs, on a constaté que la composition présentait une excellente stabilité dans le temps, le composé actif présent permettant de la protéger des phénomènes d'oxydation.

Dans cette composition, le composé de l'exemple 1 peut être avantageusement remplacé par la même quantité d'un des composés des exemples 2 et 3.

## Revendications

1. Composés de N,N'-di(aralkyl) N,N'-di(2-azaaralkyl) alkylène diamine caractérisés par le fait qu'ils correspondent à la formule générale suivante : 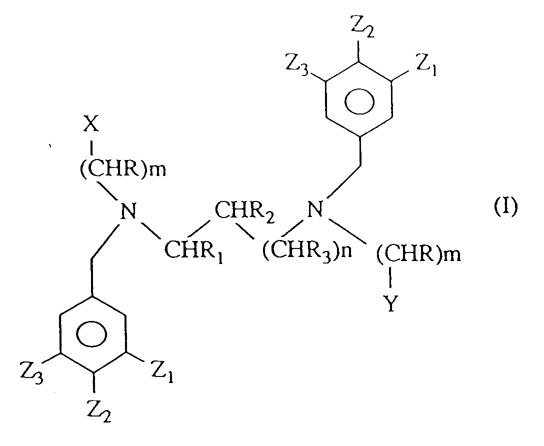 dans laquelle :
n est 0, 1 ou 2,
m est 1, 2 ou 3,
R, R₁, R₂ et R₃, identiques ou différents, représentent un atome d'hydrogène ou un radical alkyle, linéaire ou ramifié en C₁-C₄,
R₁ et R₂ ou R₂ et R₃ pris ensemble pouvant former un cycle cyclopentyle ou cyclohexyle,
X et Y identiques ou différents représentent un hétérocycle aromatique choisi parmi les radicaux 2-pyridyle, 2-pyrimidyle, 4-pyrimidyle, 1,3,5-triazin-2-yle, 2-imidazolyle, 2-pyrrolyle, 2-tétrazolyle, 4-thiazolyle, 2-méthyl-4-thiazolyle et 2-benzoimidazolyle,
Z₁, Z₂ et Z₃, identiques ou différents, représentent un atome d'hydrogène, un radical alkyle linéaire ou ramifié en C₁-C₄, le radical -OR₄ ou -NR₄R'₄,
R₄ et R'₄ représentant un atome d'hydrogène ou un radical alkyle, linéaire ou ramifié, en C₁-C₄.
et leurs sels et complexes métalliques.

2. Composés selon la revendication 1, caractérisés par le fait que le radical alkyle, linéaire ou ramifié est choisi parmi les radicaux méthyle, éthyle, isopropyle et tert-butyle.

3. Composés selon la revendication 1, caractérisés par le fait que l'un au moins des radicaux Z₁, Z₂ et Z₃ représente un groupe méthoxy.

4. Composés selon l'une quelconque des revendications précédentes, caractérisés par le fait que les sels sont des sels d'addition d'un acide minéral choisi parmi l'acide sulfurique, l'acide chlorhydrique, l'acide nitrique et l'acide phosphorique.

5. Composés selon l'une quelconque des revendications 1 à 3, caractérisés par le fait que les complexes sont des complexes formés par addition de chlorure de zinc ou de chlorure de calcium.

6. Composés selon l'une quelconque des revendications précédentes, caractérisés par le fait qu'ils sont choisis parmi :
N,N'-bis-(3,4,5-triméthoxybenzyl) N,N'-bis[1-(2-tétrazolyl) éthyl] éthylènediamine,
N,N'-bis-(3,4,5-triméthoxybenzyl) N,N'-bis-(2-pyridyl méthyl) éthylènediamine,
N,N'-bis-(2-méthyl-thiazol-4-ylméthyl)-N-N'-bis-(3,4,5,-triméthoxybenzyl) éthylènediamine,
N,N'-bis-(1H-benzoimidazol-2-ylméthyl)-N,N'-bis-(3,4,5-triméthoxybenzyl) éthylènediamine.

7. Composition pharmaceutique ou cosmétique caractérisée par le fait qu'elle contient dans un véhicule pharmaceutiquement ou cosmétiquement acceptable au moins un composé de formule (I) tel que revendiqué selon l'une quelconque des revendications 1 à 6.

8. Composition selon la revendication 7, caractérisée par le fait qu'elle contient le composé de formule (I) en une proportion de 0,001 à 10 % en poids par rapport au poids total de la composition.

9. Composition selon la revendication 7 ou 8, caractérisée par le fait qu'elle contient en outre au moins une substance active choisie parmi les antilipoperoxydants, les réducteurs biologiques, les quencheurs d'oxygène, les enzymes, une superoxyde dismutase (SOD) ou une SOD-like, la glutathion peroxydase ou des systèmes à base de sélénium.

10. Utilisation d'un composé de formule (I) selon l'une quelconque des revendications 1 à 6, pour la préparation d'une composition pharmaceutique destinée au traitement de situations de stress oxydant liées à certains états pathologiques.

11. Utilisation d'un composé de formule (I) selon l'une quelconque des revendications 1 à 6 pour la préparation d'une composition cosmétique et/ou pharmaceutique destinée à prévenir le vieillissement cutané.

12. Utilisation d'un composé de formule (I) selon l'une quelconque des revendications 1 à 6 en tant qu'agent anti-oxydant dans des compositions pharmaceutiques et cosmétiques.

## Claims

1. N,N'-Di(aralkyl)-N,N'-di(2-azaaralkyl)alkylenediamine compounds, characterized in that they correspond to the following general formula: 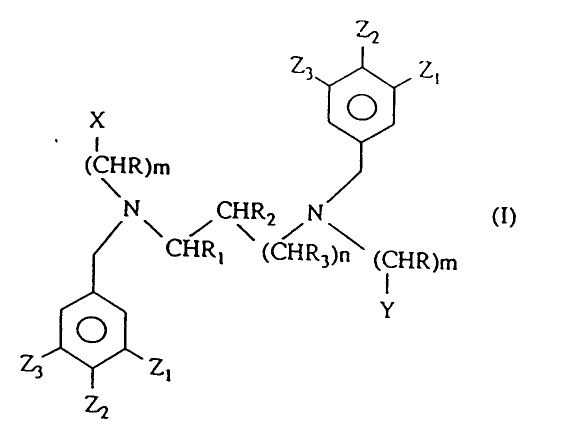 in which:
n is 0, 1 or 2,
m is 1, 2 or 3,
R, R₁, R₂ and R₃, which may be identical or different, represent a hydrogen atom or a linear or branched C₁-C₄ alkyl radical,
it being possible for R₁ and R₂ or R₂ and R₃, taken together, to form a cyclopentyl or cyclohexyl ring,
X and Y, which may be identical or different, represent an aromatic heterocycle chosen from 2-pyridyl, 2-pyrimidinyl, 4-pyrimidinyl, 1,3,5-triazin-2-yl, 2-imidazolyl, 2-pyrrolyl, 2-tetrazolyl, 4-thiazolyl, 2-methyl-4-thiazolyl and 2-benzimidazolyl radicals,
Z₁, Z₂ and Z₃, which may be identical or different, represent a hydrogen atom, a linear or branched C₁-C₄ alkyl radical, the radical -OR₄ or -NR₄R'₄,
R₄ and R'₄ representing a hydrogen atom or a linear or branched C₁-C₄ alkyl radical,
and their salts and metal complexes.

2. Compounds according to Claim 1, characterized in that the linear or branched alkyl radical is chosen from methyl, ethyl, isopropyl and tert-butyl radicals.

3. Compounds according to Claim 1, characterized in that at least one of the radicals Z₁, Z₂ and Z₃ represents a methoxy group.

4. Compounds according to any one of the preceding claims, characterized in that the salts are addition salts of an inorganic acid chosen from sulphuric acid, hydrochloric acid, nitric acid and phosphoric acid.

5. Compounds according to any one of Claims 1 to 3, characterized in that the complexes are complexes formed by addition of zinc chloride or calcium chloride.

6. Compounds according to any one of the preceding claims, characterized in that they are chosen from:
N,N'-bis(3,4,5-trimethoxybenzyl)-N,N'-bis[1-(2-tetrazolyl)ethyl]ethylenediamine,
N,N'-bis(3,4,5-trimethoxybenzyl)-N,N'-bis(2-pyridylmethyl)ethylenediamine,
N,N'-bis(2-methyl-4-thiazolylmethyl)-N,N'-bis(3,4,5-trimethoxybenzyl)ethylenediamine,
N,N'-bis(1H-benzimidazol-2-ylmethyl)-N,N'-bis-(3,4,5-trimethoxybenzyl)ethylenediamine.

7. Pharmaceutical or cosmetic composition, characterized in that it contains, in a pharmaceutically or cosmetically acceptable vehicle, at least one compound of formula (I) as claimed according to any one of Claims 1 to 6.

8. Composition according to Claim 7, characterized in that it contains the compound of formula (I) in a proportion of from 0.001 to 10% by weight relative to the total weight of the composition.

9. Composition according to Claim 7 or 8, characterized in that it also contains at least one active substance chosen from anti-lipoperoxidizing agents, biological reducing agents, oxygen quenchers, enzymes, a superoxide dismutase (SOD) or an SOD-like enzyme, glutathione peroxidase or selenium-based systems.

10. Use of a compound of formula (I) according to any one of Claims 1 to 6, for the preparation of a pharmaceutical composition intended for the treatment of oxidative stress conditions associated with certain pathological states.

11. Use of a compound of formula (I) according to any one of Claims 1 to 6, for the preparation of a cosmetic and/or pharmaceutical composition intended for preventing ageing of the skin.

12. Use of a compound of formula (I) according to any one of Claims 1 to 6, as an antioxidant in pharmaceutical and cosmetic compositions.

## Patentansprüche

1. N,N'-Di-(aralkyl)-N,N'-di-(2-aza-alkyl)-alkylendiamin-Verbindungen, gekennzeichnet durch folgende allgemeine Formel: 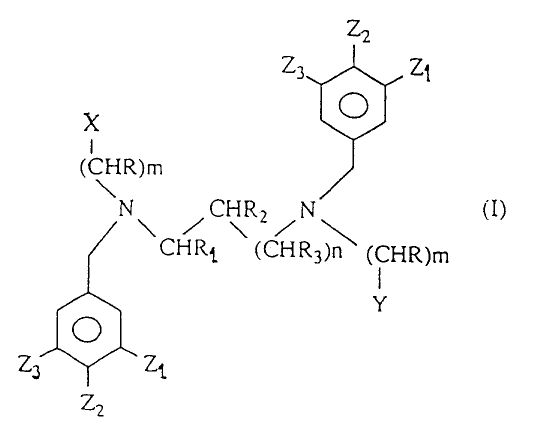 worin
n 0, 1 oder 2 und
m 1, 2 oder 3 bedeuten,
R, R₁, R₂ und R₃, die gleich oder verschieden sind, ein Wasserstoffatom oder einen gerad- oder verzweigtkettigen C₁-C₄- Alkylrest bedeuten,
R₁, R₂, R₂ und R₃ zusammengenommen einen Cyclopentyl- oder Cyclohexylring bilden können,
X und Y, die gleich oder verschieden sind, einen aromatischen Heterocyclus darstellen, der aus den Gruppen 2-Pyridyl, 2-Pyrimidyl, 4-Pyrimidiyl, 3,5-Triazinyl, 2-Imidazolyl, 2-Pyrrolyl, 2-Tetrazolyl, 4-Thiazolyl, 2-Methyl-4-thiazolyl sowie 2-Benzimidazolyl ausgewählt ist.
Z₁, Z₂, und Z₃, die gleich oder verschieden sind, ein Wasserstoffatom, einen gerad- oder verzweigtkettigen C₁-C₄-Alkylrest, den Rest -OR₄ oder -NR₄R'₄ bedeuten, wobei
R₄ und R'₄ ein Wasserstoffatom oder einen gerad- oder verzweigtkettigen C₁-C₄-Rest darstellen, sowie
deren Salze und Metallkomplexe.

2. Verbindungen nach Anspruch 1, dadurch gekennzeichnet, daß der gerad- oder verzweigtkettige Alkylrest unter den Gruppen Methyl, Ethyl, Isopropyl und tert-Butyl ausgewählt ist.

3. Verbindungen nach Anspruch 1, dadurch gekennzeichnet, daß mindestens einer der Reste Z₁, Z₂ und Z₃ eine Methoxygruppe bedeutet.

4. Verbindungen nach einem der vorstehenden Ansprüche, dadurch gekennzeichnet, daß die Salze Additionssalze einer Mineralsäure sind, die unter Schwefelsäure, Salzsäure, Salpetersäure und Phosphorsäure ausgewählt ist.

5. Verbindungen nach einem der Ansprüche 1 bis 3, dadurch gekennzeichnet, daß die Komplexe als Komplexverbindungen vorliegen, die durch die Addition von Zinkchlorid oder Calciumchlorid gebildet sind.

6. Verbindungen nach einem der vorstehenden Ansprüche, dadurch gekennzeichnet, daß sie unter den folgenden ausgewählt sind:
N,N'-Bis-(3,4,5-trimethoxybenzyl)-N,N'-bis[1-(2-tetrazolyl)-ethyl]ethylendiamin,
N,N'-Bis-(3,4,5-trimethoxybenzyl)-N,N'-bis-(2-pyridylmethyl)-ethylendiamin,
N,N'-Bis-(2-methylthiazol-4-yl-methyl)-N-N'-bis-(3,4,5-trimethoxybenzyl)ethylendiamin oder
N,N'-Bis-(1H-benzimidazol-2-yl-methyl)-N,N'-bis-(3,4,5-trimethoxybenzyl)ethylendiamin.

7. Pharmazeutische oder kosmetische Zubereitung, dadurch gekennzeichnet, daß sie in einem pharmazeutisch oder kosmetisch annehmbaren Träger mindestens eine Verbindung der Formel I gemäß den Ansprüchen 1 bis 6 enthält.

8. Zubereitung nach Anspruch 7, dadurch gekennzeichnet, daß sie die Verbindung der Formel (I) in einem Verhältnis von 0,001 bis 10 Gew.-%, bezogen auf das Gesamtgewicht der Zubereitung, enthält.

9. Zubereitung nach Anspruch 7 oder 8, dadurch gekennzeichnet, daß sie zusätzlich noch mindestens einen Wirkstoff enthält, der aus Antilipoperoxidantien, biologischen Reduktionsmitteln, Sauerstoffängern, Enzymen, einer Superoxid-Dismutase (SOD) oder einer SOD-artigen Substanz, der Glutathionperoxidase oder Systemen auf Basis von Selen ausgewählt ist.

10. Verwendung einer Verbindung gemäß der Formel (I) nach einem der Ansprüche 1 bis 6 zur Herstellung einer pharmazeutischen Zubereitung, die zur Behandlung von Oxidationsstreßsituationen bestimmt ist, die mit bestimmten pathologischen Zuständen in Zusammenhang stehen.

11. Verwendung einer Verbindung gemäß der Formel (I) nach einem der Ansprüche 1 bis 6 zur Herstellung einer kosmetischen und/oder pharmzeutischen Zubereitung, die zur Verhütung der Hautalterung bestimmt ist.

12. Verwendung einer Verbindung gemäß der Formel (I) nach einem der Ansprüche 1 bis 6 als Antioxidans in pharmazeutischen oder kosmetischen Zubereitungen.
